# EUROPEAN PATENT APPLICATION

(11) **EP 2 502 570 A1**
(43) Date of publication of application: **26.09.2012**
(21) Application number: 12160701.4
(22) Date of filing: 22.03.2012
(51) Int. Cl.: A61B 17/06, A61B 17/00

(54) **Welded looped suture**

(30) Priority: 23.03.2011 US 201161466673 P; 14.03.2012 US 201213419718
(71) Applicant: Tyco Healthcare Group LP, Mansfield, MA 02048 (US)
(72) Inventor: Glick, Jonathan, Southport, CT Connecticut 06890-1161 (US)
(74) Representative: Soames, Candida Jane

(57) **Abstract**

A suture includes an elongate body and a filler material. The elongate body includes a distal end having first and second overlapping sections forming a loop. The filler material is disposed within at least a portion of a seam defined between the first and second overlapping sections of the elongate body to reinforce the first and second overlapping sections. A method of forming a reinforced looped suture includes providing a suture including an elongate body including a distal end having first and second overlapping sections defining a loop, and applying a filler material to a seam defined between the first and second overlapping sections.

## Description

### CROSS-REFERENCE TO RELATED APPLICATION

This application claims the benefit of, and priority to, U.S. Provisional Application No. 61/466,673, filed on March 23, 2011, the entire disclosure of which is incorporated by reference herein.

### BACKGROUND

### Technical Field

The present disclosure relates to looped sutures and methods of forming the same. More particularly, the present disclosure relates to looped sutures including a reinforced welded joint to improve the strength of the suture and resistance to shear failure.

### Background of Related Art

Loops may be formed in a suture during, or prior to, a wound closing procedure. A loop may be formed in a suture for a number of reasons. For example, during manufacture a loop may be formed in the suture to assist in further processing of the suture, e.g., for holding the suture as barbs are formed along the length thereof. Alternatively, a loop formed in a suture during manufacture may be used to secure the suture to tissue. In this manner, once the non-looped end of the suture is inserted through tissue, that end may be threaded through the loop to form a slip knot-like configuration that may be tied to secure tissue. In another application, a loop may be formed in a suture in place of a knot. This requires the use of a handheld instrument that may be brought into an operating room for securing the first and second ends of a suture that have been received through opposing sections of tissue.

Regardless of the reason for forming the loop, when a loop is formed in a suture, whether using adhesive, heat, or ultrasonic energy, the diameter of the suture is doubled where the two suture portions overlap. In the event that the suture loop is used to secure tissue, the doubling of the diameter of the suture in order to create the loop increases the amount of force necessary to pull the loop through tissue. Therefore, it would be beneficial to have a system and method of forming a looped suture to reinforce the overlapping portion such that the strength of the suture is increased and the suture is more resistant against shear stress failure.

### SUMMARY

The present sutures include an elongate body and a filler material. The elongate body includes a distal end having first and second overlapping sections forming a loop. The filler material is disposed within at least a portion of a seam defined between the first and second overlapping sections of the elongate body to reinforce the first and second overlapping sections. The first and second overlapping sections may include a taper in a proximal end thereof. In embodiments, the suture includes a bioactive agent.

The elongate body and the filler material may be fabricated from the same or different polymeric materials. In embodiments, the filler material includes a thermoplastic polymer. In embodiments, the first and second overlapping sections of the elongate body may be substantially parallel to each other. In other embodiments, the first and second overlapping sections may be intertwined.

Methods of forming a reinforced looped suture are also described. In accordance with the present methods, a suture including an elongate body including a distal end having first and second overlapping sections defining a loop is provided, and a filler material is applied to a seam defined between the first and second overlapping sections. In embodiments, the filler material is sprayed towards the seam of the first and second overlapping sections of the elongate body. In other embodiments, a pre-molded filler may be applied to the seam of the first and second overlapping sections of the elongate body.

The filler material may be provided at a temperature that is sufficient to locally melt the first and second overlapping sections of the elongate body to create a reinforced joined segment. In embodiment in which the filler material is the same as the material forming the first and second overlapping portions, a homogeneous joined segment is formed. In embodiments, the filler material may be welded to the first and second overlapping sections to create the reinforced joined segment. Welding may be accomplished by the application of radiofrequency (RF), ultrasonic, laser, electric arc discharge, or thermal energy.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1A is a side view of a looped suture in accordance with an embodiment of the present disclosure;

FIG. 1B is a cross-sectional end view of the looped suture of FIG. 1A, taken along line 1B-1B;

FIG. 2A is a schematic illustration of a method of applying filler material to a looped suture in accordance with an embodiment of the present disclosure;

FIG. 2B is a schematic illustration of a method of applying filler material to a looped suture in accordance with another embodiment of the present disclosure;

FIGS. 3A and 3B are cross-sectional views of a looped suture in a welding assembly in a pre-welded (FIG. 3A) and a post-welded (FIG. 3B) configuration in accordance with an embodiment of the present disclosure;

FIG. 4A is a side view of a looped suture in accordance with another embodiment of the present disclosure; and

FIG. 4B is a side view of the looped suture of FIG. 4A including a filler material.

### DETAILED DESCRIPTION

A looped suture and method of forming the same are described herein. Various exemplary embodiments of the present disclosure are discussed hereinbelow in terms of a welded looped suture. A looped suture in accordance with the present disclosure includes a first section of suture which is joined with a second section of suture to form a loop. The adjacent surfaces of the first and second sections are reinforced to increase the strength at the junction of the first and second sections of the suture.

In the following discussion, the term "proximal" should be understood as referring to the portion of a structure that is closer to a clinician during proper use. The term "distal" should be understood as referring to the portion of a structure that is further from the clinician during proper use.

The following discussion includes a description of embodiments of the presently disclosed looped suture, as well as a description of exemplary corresponding methods of forming the looped suture and using the looped suture in accordance with the principles of the present disclosure.

Looped sutures described herein may be formed from any sterilizable biocompatible material that has suitable physical properties for the intended use of the suture. The sutures described herein may be monofilament or multifilament sutures formed from natural, synthetic, degradable, and/or non-degradable materials, as well as combinations thereof. The sutures may be formed from biocompatible polymers, such as homopolymers or copolymers, including random copolymers, block copolymers, or graft copolymers. The biocompatible polymers may be linear polymers, branched polymers, or dendrimers.

Representative degradable polymers which may be utilized to form the suture include: polysaccharides such as alginate, dextran, chitin, chitosan, hyaluronic acid, cellulose, collagen, gelatin, fucans, glycosaminoglycans, and chemical derivatives thereof (substitutions and/or additions of chemical groups include, for example, alkyl, alkylene, amine, sulfate, hydroxylations, carboxylations, oxidations, and other modifications routinely made by those skilled in the art); catgut; silk; linen; cotton; proteins such as albumin, casein, zein, silk, and soybean protein; polyhydroxy acids prepared from lactone monomers such as glycolide, lactide, caprolactone, ε-caprolactone, valerolactone, and δ-valerolactone, carbonates (e.g., trimethylene carbonate, tetramethylene carbonate, and the like), dioxanones (e.g., 1,4-dioxanone and p-dioxanone), and 1,dioxepanones (e.g., 1,4-dioxepan-2-one and 1,5-dioxepan-2-one); poly(hydroxyalkanoate)s such as polyhydroxybutyrate, polyhydroxyvalerate, poly(3-hydroxybutyrate-co-3-hydroxyvalerate), polyhydroxyoctanoate, and polyhydroxyhexanoate; polyalkylene oxalates; polyoxaesters; polyanhydrides; polyester anhydrides; polyortho esters; and copolymers, block copolymers, homopolymers, blends, and combinations thereof.

Suitable non-degradable materials which may be utilized to form the sutures include polyolefins such as polyethylene (including ultra high molecular weight polyethylene) and polypropylene including atactic, isotactic, syndiotactic, and blends thereof; polyethylene glycols; polyethylene oxides; polyisobutylene and ethylene-alpha olefin copolymers; fluorinated polyolefins such as fluoroethylenes, fluoropropylenes, fluoroPEGSs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polyether-esters such as polybutester; polytetramethylene ether glycol; 1,4-butanediol; polyurethanes; acrylic polymers; methacrylics; vinyl halide polymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; ethylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; carbon fibers; and copolymers and combinations thereof.

The sutures may be formed using any technique within the purview of those skilled in the art such as, for example, extrusion, molding, casting, and/or spinning. Looped sutures may also be formed by tying a noose, cinch, or knot in a suture. Looped sutures may also be created through use of laser or ultrasonic welding, or through use of degradable or permanent glues or adhesives. In some embodiments, the sutures may include a yarn made of more than one filament, which may contain multiple filaments of the same or different materials. Where the suture is made of multiple filaments, the suture may be made using any known technique such as, for example, braiding, weaving or knitting. The sutures may also be drawn, oriented, annealed, calendared, crinkled, twisted, commingled, or air entangled to form yarns as part of the suture forming process.

Although shown having a circular cross-sectional geometry, the cross-sectional geometry of the suture may be of any suitable shape such as, round, elliptical, square, flat, octagonal, and rectangular.

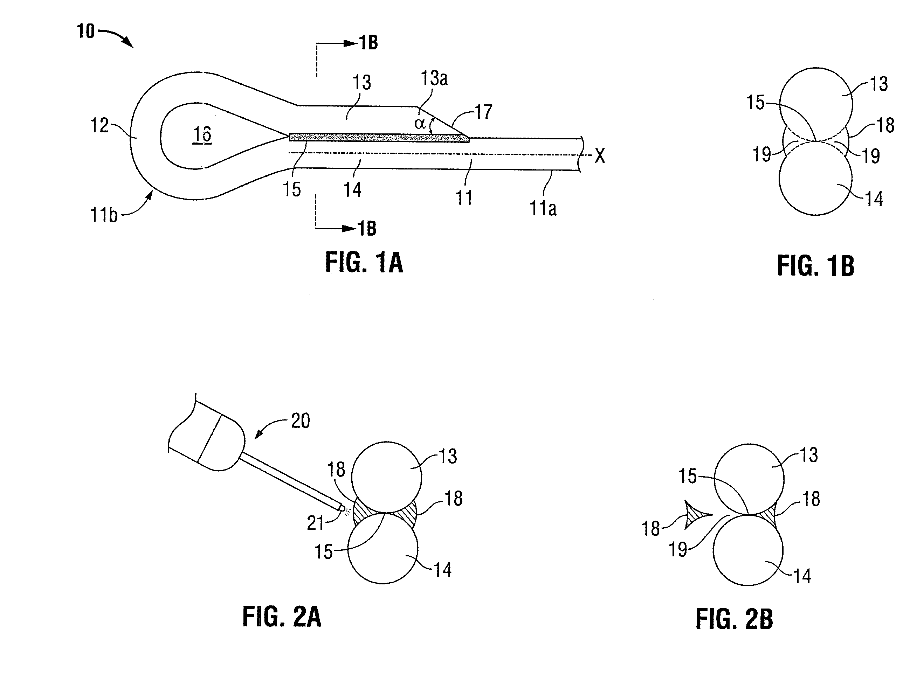

Referring now to the drawings, FIGS. 1A and 1B illustrate a looped suture of the present disclosure. Looped suture 10 includes elongate body 11 having proximal end 11a and distal end 11b. Proximal end 11a of elongate body 11 may be attached to one or more suture needles (not shown). Distal end 11b of elongate body 11 includes first section 13 overlaying second section 14 to form loop 12. The adjacent surfaces of first and second sections 13, 14 form a joined segment or joint 15. As illustrated in the present embodiment, loop 12 forms a substantially tear drop shape defining a distal opening 16. Loop 12 may also be formed in any size. For example, in embodiments, loop 12 is sized to receive proximal end 11a of looped suture 10.

First and second sections 13, 14 of elongate body 11 of looped suture 10 may be welded together to form joined segment 15. Energy is locally applied to first and second sections 13, 14 of elongate body 11 fusing sections 13, 14 together to form joined segment 15. Various types of energy may be applied to first and second sections 13, 14 to form joined segment 15, including RF, ultrasonic, laser, electrical arc discharge, and thermal. Alternatively, first and second sections 13, 14 of elongate body 11 may be joined using glue, epoxy or other adhesives. In yet other embodiments, first and second sections 13, 14 of elongate body 11 may simply be positioned tangentially to, or touching, one another until the application of the filler material, as described in further detail below.

A proximal end 13a of first section 13 may be angled to form a tapered surface 17. Tapered surface 17 angles downwardly towards proximal end 11a of elongate body 11 of looped suture 10. Tapered surface 17 may form an angle "α" relative to a longitudinal axis "X" of second section 14, between zero degrees (0°) and ninety degrees (90°). Tapered surface 17 facilitates insertion of loop 12 into or through tissue. Tapered surface 17 may be formed prior to, during, or following the joining of first and second sections 13, 14. Tapered surface 17 forms a smooth transition with second section 14 of elongate body 11 for ease of insertion through tissue and to decrease the shear stress applied to the first and second sections 13, 14 as looped suture 10 is pulled through tissue.

Although shown having a substantially planar taper, tapered surface 17 may include any number of configurations. For example, tapered surface 17 may be beveled, may include a laterally and longitudinally concave taper, may include a laterally and longitudinally convex taper, or may include any combination thereof. Tapered surface 17 may be selected depending on the tissue being sutured and/or the depth loop 12 is desired to be received within the tissue.

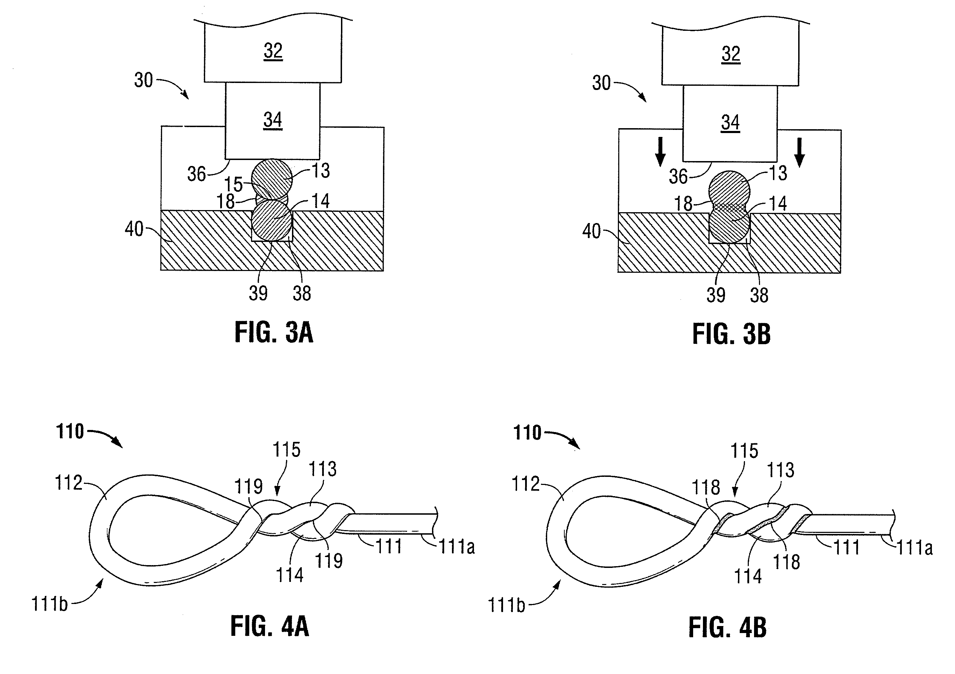

An example of a process of forming a loop 12 is described by Nicholas Maiorino et al. in U.S. Patent Publication No. 2010/0071833, the entire contents of which is herein incorporated by reference. Briefly, this method involves utilizing a system including a base, a suture retaining member, a suture tensioning member, a welding assembly, and a cutting assembly. In this system, proximal end 11a of elongated body 11 is securely locked in a clamp, second section 14 of elongate body 11 is positioned within a channel in the base, and elongate body 11 is wrapped around a pin before first section 13 is placed on top of, or adjacent, second section 14. A distal end 11b of elongate body 11 is then received in a tension cylinder. Once first and second sections 13, 14 are positioned adjacent one another, a welding assembly is approximated towards the suture retaining member to melt the contacting portions between first and second sections 13, 14 to create joined segment 15. The welding assembly may be an ultrasonic welding assembly as described in detail below with reference to FIGS. 3A and 3B. After first and second sections 13, 14 are fused to create joined segment 15, the welding assembly may be approximated away from the suture retaining member and replaced or exchanged for cutting assembly to complete the tapered cutting of proximal end 13a of first section 13.

Other methods of forming a looped suture are within the purview of those skilled in the art, such as processes described by Nicholas Maiorino et al. in U.S. Patent Publication Nos. 2010/0101707 and 2010/0276062, the entire contents of which are herein incorporated by reference.

A reinforcement or filler material 18 is applied to the first and second sections 13, 14 about joined segment 15. Filler material 18 is applied to the seam 19 created between the first and second sections 13, 14 to increase the strength of the joined segment 15. Filler material 18 is a biocompatible polymeric material, or composite thereof, that may be the same or different than the material from which the looped suture 10 is fabricated. The filler material 18 reinforces the joined segment 15 by increasing the strength thereof, which may be related to increasing the thickness of the joined segment 15. In embodiments, the filler material 18 may be a thermoplastic polymer that can be heated to a soft and/or molten state, and subsequently cooled to return the filler material 18 to a solid state.

The filler material 18 may be applied to the joined segment 15 utilizing techniques known to one skilled in the art, e.g., dipping, wiping, spraying, etc. For example, the filler material 18 may be applied by spraying a solution or melt of the filler material 18 from the tip 21 of an applicator 20 towards the joined segment 15 as illustrated in FIG. 2A. The filler material 18 may provide a strong interface between the first and second sections 13, 14 of the suture and improve the filling of voids between the first and second sections 13, 14. In other embodiments, the filler material 18 may be a solid polymer that is extruded or molded to fit within the seam 19 between first and second sections 13, 14 as illustrated in FIG. 2B. The filler material 18 may be attached to the seam using adhesives, epoxies, or glues. Conversely, the filler material 18 may be attached to the seam utilizing various energy forms described herein, including, but not limited to, thermal, ultrasonic and laser.

In embodiments, filler material 18 may be provided at a temperature that is sufficient to locally melt the first and second sections 13, 14 of elongate body 11 and create a solid structure (FIG. 1B). In embodiments in which the filler material 18 is the same as the material forming the first and second sections 13, 14, a homogeneous joined segment 15 is produced. In other embodiments, the filler material 18 is welded to the first and second sections 13, 14 of the elongate body 11 to provide the solid joined segment 15. In such embodiments, welding may be achieved via RF, ultrasonic, laser, electrical arc discharge, and thermal energy as discussed above.

With reference to FIGS. 3A and 3B, an ultrasonic welding process is performed after the filler material 18 is applied to joined segment 15. Welding assembly 30 includes an ultrasonic device 32 operably connected to a generator (not shown) for ultrasonically vibrating a die 34 extending from ultrasonic device 32. Die 34 defines substantially flat suture contacting portion 36. In an alternative embodiment, die 34 may include a channel (not shown) including a suture contacting portion (not shown) configured to receive first section 13 of elongate body 11. The channel and suture contacting portion of the die 34 may be substantially the same as the channel 38 and the suture contacting portion 39 defined within base 40. Suture contacting surface 39 may define a substantially planar surface or may instead form a surface corresponding to the contour of elongate body 11. Thus, suture contacting portion 39 may include a concave, convex or beveled surface to correspond with an elongate body 11 having a convex, concave or beveled profile. In one embodiment, welding assembly 30 is operatively mounted on a press assembly (not shown) for approximating die 34 of welding assembly 30 towards and away from base 40. Alternatively, welding assembly 30 may be securely mounted relative to base 40 and base 40 may be raised and lowered to approximate base 40 towards and away from die 34. The downward pressure exerted on first and second sections 13, 14, indicated by the arrows in FIG. 3B, and the ultrasonic vibration of die 34 causes the portions of the first section 13, second section 14, and filler material 18 that are in contact with each other to locally heat, and in some instances, begin to melt thereby forming joined segment 15 which is reinforced with filler material 18.

Referring now to FIGS. 4A and 4B, a looped suture 110 in accordance with another embodiment of the present disclosure is provided. Suture 110 includes elongate body 111 having proximal end 111a and distal end 111b. Proximal end 111a of elongate body 111 may be attached to one or more suture needles (not shown). Distal end 111b of elongate body 111 includes first section 113 and second section 114 which are twisted, braided, or otherwise intertwined to form joined segment 115 defining loop 112. The first and second sections 113, 114 may be twisted a prescribed number of revolutions at a controlled or variable pitch.

Filler material 118 may be applied to the twisted joined segment 115 along the curved seams 119 formed between the first and second sections 113, 114 and optionally, be welded thereafter. In embodiments, the first and second sections 113, 114 of the joined segment 115 may be ultrasonically welded without the use of filler material 118. Twisting of the first and second sections 113, 114 changes the direction of force required for the suture to fail in shear from a constant pull along the axis of the suture when the first and second sections 13, 14 are aligned substantially parallel with each other (FIGS. 1A-3B) to a helical path around the axis of the suture (FIGS. 4A-4B).

In embodiments, at least one bioactive agent may be combined with or applied on at least a portion of suture 10 described herein. For example, a bioactive agent may be combined with the polymer used to form the suture, and/or a bioactive agent may be applied as a continuous or discontinuous coating covering at least a portion of a surface thereof. Bioactive agents may be applied onto the suture utilizing any method within the purview of one skilled in the art including, for example, spraying, dipping, brushing, rolling, wiping, painting, extruding, and the like. The at least one agent may be freely released by the suture or may be chemically bound to the surface of the suture.

Bioactive agents include substances which are beneficial and tend to promote the healing process. For example, the looped sutures can be provided with a bioactive agent that will be deposited at the sutured site. The bioactive agent can be chosen for its antimicrobial properties, capability for promoting wound repair and/or tissue growth, or for specific indications such as thrombosis. In embodiments, combinations of such agents may be applied to the medical device of the present disclosure before, during, or after suture formation.

Suitable bioactive agents include, for example, biocidal agents, antimicrobial agents, antibiotics, anti-proliferatives, medicants, growth factors, anti-clotting agents, clotting agents, analgesics, anesthetics, anti-inflammatory agents, wound repair agents and the like, chemotherapeutics, biologics, protein therapeutics, monoclonal or polyclonal antibodies, DNA, RNA, peptides, polysaccharides, lectins, lipids, probiotics, diagnostic agents, angiogenics, anti-angiogenic drugs, polymeric drugs, and combinations thereof.

Although the above bioactive agents have been provided for the purposes of illustration, it should be understood that the present disclosure is not so limited. In particular, although certain bioactive agents are specifically referred to above, the present disclosure should be understood to include analogues, derivatives, and conjugates of such agents.

The sutures may be dyed in order to increase the visibility of the suture in the surgical field. Any dye suitable for incorporation in medical devices may be used. Such dyes include, but are not limited to, carbon black, bone black, FD&C Blue #1, FD&C Blue #2, FD&C Blue #3, FD&C Blue #6, D&C Green #6, D&C Violet #2, methylene blue, indocyanine green, other colored dyes, and combinations thereof. It is envisioned that visualization agents may also be used, such as fluorescent compounds (e.g., fluorescein or eosin), x-ray contrast agents (e.g., iodinated compounds), ultrasonic contrast agents, and MRI contrast agents (e.g., Gadolinium containing compounds).

In use, the looped sutures described herein may include a needle (not shown) on the proximal end thereof. The needle is inserted into and through a first and second flap of tissue. The looped suture may be pulled through the tissue until the proximal end of the first overlapping section contacts the tissue. Once a portion of the loop of the suture is received within the tissue, the proximal end of the suture may be inserted through the loop. The proximal end of the suture may then be pulled tight, thereby approximating the first and second tissue flaps towards one another. The proximal end of the suture may then be knotted or otherwise secured to the loop. In one embodiment, a knot may be formed in the proximal end to prevent the proximal end from withdrawing from the loop. In another embodiment, the proximal end of the suture may be tied directly to the loop.

Persons skilled in the art will understand that the devices and methods specifically described herein, and illustrated in the accompanying drawings, are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosed devices and methods based on the above-described embodiments. As such, further modifications and equivalents of the invention herein disclosed can occur to persons skilled in the art using no more than routine experimentation, and all such modifications and equivalents are believed to be within the spirit and scope of the disclosure as defined by the following paragraphs.

The invention may be described by reference to the following numbered paragraphs:-

A suture comprising:
an elongate body including a distal end having first and second overlapping sections forming a loop; and
a filler material disposed within at least a portion of a seam defined between the first and second overlapping sections of the elongate body.

The suture of paragraph [0045], wherein the filler material reinforces the first and second overlapping sections.

The suture of paragraph [0045] wherein the elongate body and the filler material comprise a same polymeric material.

The suture of paragraph [0045], wherein the elongate body and the filler material comprise different polymeric materials.

The suture of paragraph [0045], wherein the filler material comprises a thermoplastic polymer.

The suture of paragraph [0045], wherein the first and second overlapping sections further comprise a taper in a proximal end thereof.

The suture of paragraph [0045] wherein the first and second overlapping sections are substantially parallel to each other.

The suture of paragraph [0045], wherein the first and second overlapping sections are intertwined.

The suture of paragraph [0045], further comprising a bioactive agent.

A method of forming a reinforced looped suture, the method comprising the steps of: providing a suture including an elongate body including a distal end having first and second overlapping sections defining a loop; and applying a filler material to a seam defined between the first and second overlapping sections.

The method of paragraph [0054], wherein the step of applying the filler material further comprises the step of spraying the filler material towards the seam of the first and second overlapping sections of the elongate body.

The method of paragraph [0054], wherein the step of applying the filler material further comprises the step of applying a pre-molded filler within the seam of the first and second overlapping sections of the elongate body.

The method of paragraph [0054], wherein the step of applying the filler material further comprises the step of providing the filler material at a temperature that is sufficient to locally melt the first and second overlapping sections of the elongate body to create a reinforced joined segment.

The method of paragraph [0057], wherein the step of applying the filler material further comprises applying a filler material comprising a same material as the first and second overlapping sections to form a homogenous joined segment.

The method of paragraph [0054], further comprising the step of welding the filler material to the first and second overlapping sections to create a reinforced joined segment.

The method of paragraph [0059], wherein the step of welding is accomplished by application of one of RF, ultrasonic, laser, electrical arc discharge, and thermal energy to the looped suture.

## Claims

1. A suture comprising:
an elongate body including a distal end having first and second overlapping sections forming a loop; and
a filler material disposed within at least a portion of a seam defined between the first and second overlapping sections of the elongate body.

2. The suture of claim 1, wherein the filler material reinforces the first and second overlapping sections.

3. The suture of claim 1 or claim 2, wherein the elongate body and the filler material comprise a same polymeric material; or wherein the elongate body and the filler material comprise different polymeric materials.

4. The suture of any preceding claim, wherein the filler material comprises a thermoplastic polymer.

5. The suture of any preceding claim, wherein the first and second overlapping sections further comprise a taper in a proximal end thereof.

6. The suture of any preceding claim, wherein the first and second overlapping sections are substantially parallel to each other.

7. The suture of any of claims 1 to 5, wherein the first and second overlapping sections are intertwined.

8. The suture of any preceding claim, further comprising a bioactive agent.

9. A method of forming a reinforced looped suture, the method comprising the steps of:
providing a suture including an elongate body including a distal end having first and second overlapping sections defining a loop; and
applying a filler material to a seam defined between the first and second overlapping sections.

10. The method of claim 9, wherein the step of applying the filler material further comprises the step of spraying the filler material towards the seam of the first and second overlapping sections of the elongate body.

11. The method of claim 9 or claim 10, wherein the step of applying the filler material further comprises the step of applying a pre-molded filler within the seam of the first and second overlapping sections of the elongate body.

12. The method of any of claims 9 to 11, wherein the step of applying the filler material further comprises the step of providing the filler material at a temperature that is sufficient to locally melt the first and second overlapping sections of the elongate body to create a reinforced joined segment.

13. The method of claim 12, wherein the step of applying the filler material further comprises applying a filler material comprising a same material as the first and second overlapping sections to form a homogenous joined segment.

14. The method of any of claims 9 to 13, further comprising the step of welding the filler material to the first and second overlapping sections to create a reinforced joined segment.

15. The method of claim 14, wherein the step of welding is accomplished by application of one of RF, ultrasonic, laser, electrical arc discharge, and thermal energy to the looped suture.
